# EUROPEAN PATENT SPECIFICATION

(11) **EP 2 510 923 B1**
(45) Date of publication and mention of the grant of the patent: **31.12.2014**
(21) Application number: 12160977.0
(22) Date of filing: 23.03.2012
(51) Int. Cl.: A61K 9/28, C09D 101/26, C09D 101/28

(54) **Coating composition, solid preparation coated therewith, and method for preparing solid preparation**
Beschichtungszusammensetzung, damit beschichtete Feststoffzubereitung, und Verfahren zur Herstellung der Feststoffzubereitung
Composition de revêtement, préparation solide revêtue avec celle-ci et procédé de préparation de la formulation solide

(30) Priority: 30.03.2011 JP 2011075344
(43) Date of publication of application: 17.10.2012
(73) Proprietor: Shin-Etsu Chemical Co., Ltd., Tokyo 100-0004 (JP)
(72) Inventor: Fukasawa, Miyuki, JOETSU-SHI, Niigata 942-8601 (JP); Nishiyama, Yuichi, JOETSU-SHI, Niigata 942-8601 (JP); Hoshino, Takafumi, JOETSU-SHI, Niigata 942-8601 (JP)
(74) Representative: Larcher, Dominique

(56) References cited:
- EP-A1- 1 473 030
- WO-A2-2004/041244
- MARUCCI M ET AL: "Coated formulations: New insights into the release mechanism and changes in the film properties with a novel release cell", JOURNAL OF CONTROLLED RELEASE, ELSEVIER, AMSTERDAM, NL, vol. 136, no. 3, 19 June 2009 (2009-06-19) , pages 206-212, XP026142714, ISSN: 0168-3659, DOI: 10.1016/J.JCONREL.2009.02.017 [retrieved on 2009-02-27]

## Description

### BACKGROUND OF THE INVENTION

### 1. Field of the Invention

The present invention relates to a coating composition enabling delayed release of a drug, a solid preparation coated therewith, and a method for preparing the solid preparation.

### 2. Description of the Related Art

In recent years, it has been elucidated that the pharmacokinetics or pharmacological effect of drugs has time dependence so that various drug delivery systems have been proposed in order to cause the drugs to act on diseased sites accurately. As oral drugs, delayed release preparations, sustained release preparations, and the like are known.

With respect to such delayed release preparations, there are disclosed a controlled release preparation which is obtained by coating a drug-containing core with an aqueous dispersion of two or more acrylic polymers, and which controls the pH for dissolving the coating film to adjust the release of a drug (JP 08-143476A), a method of coating a core containing a drug and a disintegrant with a dispersion of a water-insoluble polymer to obtain a preparation having a lag time (JP 2009-191034A), and a method of coating a core containing a drug and a disintegrant with a solution containing an inorganic substance and a water-insoluble polymer to obtain a preparation having a lag time (JP 2000-128779A). In addition, sugar coating is also known as a coating method for obtaining a preparation having a lag time. Further, examples of a composition for a coating solution obtained by mixing a water-soluble cellulose ether with a cellulose-based enteric base material include a composition used for an oral sustained release preparation (JP 2006-507298T, which is a national phase publication of WO 2004/041244) and a composition for a coating solution to be used for a delayed release portion of a combined preparation (JP 2010-505943T, which is a national phase publication of WO 2008/044862).

### SUMMARY OF THE INVENTION

In the method of using an acrylic polymer as described in JP 08-143476A, the time at which the preparation starts releasing a drug is not controlled, but the dissolution by pH of the solution in which the preparation dissolves is controlled. Due to an individual variability in pH level of a gastric juice or the like, it is very difficult to accurately adjust the pharmacokinetics. The coating of a water-insoluble polymer dispersion as shown in JP 2009-191034A cannot provide a uniform film so that it is very difficult to control a lag time (in which a preparation does not release a drug) and a drug release property after a lag time. Further, since the coating of not a polymer solution but a polymer dispersion does not provide a uniform film, a stable lag time cannot be ensured. The coating of a solution containing an inorganic substance and a water-insoluble polymer as shown in JP 2000-128779A requires a large amount of the inorganic substance for forming a film so that there is concern of such a substance causing an uncomfortable texture during dosage or of poor workability during coating. Accordingly, there has been a strong demand for the development of a delayed release preparation which can be prepared easily and does not require an advanced pharmaceutical formulation technology.

On the other hand, JP 2006-507298T, which is a national phase publication of WO 2004/041244, discloses a sustained release method of adding a water-soluble cellulose ether in an amount of about 5% by weight to an enteric base material to form pores. However, the resulting mixture contains the enteric base material at an excessive ratio so that prompt dissolution of a drug in the stomach cannot be attained.

JP 2010-505943T, which is a national phase publication of WO 2008/044862, discloses a method in which a water-insoluble polymer, an enteric polymer and a water-soluble polymer are added to a delayed dissolution portion and the resulting mixture is combined with an immediate dissolution portion to form delayed immediate release. Since the amount of the water-soluble polymer added is as small as about 10% by weight, prompt dissolution of a drug in the stomach cannot be attained.

Sugar coating can provide a stable lag time and stable dissolution immediately after preparation. However, a protein, which is a main component of gelatin used as a binder, denatures so that there is a problem that the dissolution delays as time goes by. Accordingly, a coating method which does not cause a time-dependent change in dissolution has been demanded.

An object of the invention is to provide a coating composition for a delayed release preparation which does not undergo a time-dependent change and can release a drug promptly in the stomach after a time (lag time) in which the solid preparation does not release the drug; a solid preparation coated with the coating composition; and a method for preparing the solid preparation.

The present inventors have investigated extensively with a view to preparing a coating composition free from a time-dependent change and capable of providing a uniform film by a simple method, and developing a delayed release preparation capable of releasing a drug promptly in the stomach after a lag time. As a result, it has been found that the above-described object can be achieved only by covering a drug-containing core with a coating composition comprising at least a nonionic water-soluble cellulose ether and a cellulose-based enteric base material, leading to the completion of the invention.

According to the invention, there is provided a coating composition comprising at least a nonionic water-soluble cellulose ether and a cellulose-based enteric base material wherein a weight ratio of the nonionic water-soluble cellulose ether to the cellulose-based enteric base material is from 95:5 to 65:35. There is also provided a solid preparation comprising at least a drug-containing core and the coating composition which covers the core wherein the drug dissolves promptly in a stomach after a lag time. In other words, a time-delayed preparation is provided. There is further provided a method for preparing a solid preparation comprising at least the steps of applying, to a drug-containing core, a solution of the coating composition in a solvent; and drying to remove the solvent.

According to the invention, a drug-containing core is covered with a coating composition comprising at least a nonionic water-soluble cellulose ether and a cellulose-based enteric base material so that the drug with a lag time can be released and prompt dissolution of the drug in the stomach after the lag time can be attained. In addition, the lag time can be controlled by changing the composition or the coating amount of the coating composition. Further, the composition having a controlled lag time can be used as an alternative of sugar coating.

### BRIEF DESCRIPTION OF THE DRAWINGS

FIG. 1 shows the results of the dissolution tests of Examples 1 to 3, 6 and 10;
FIG. 2 shows the results of the dissolution tests of Examples 4, 5 and 7 to 9;
FIG. 3 shows the results of the dissolution tests of Comparative Examples 1 to 4;
FIG. 4 shows the results of the dissolution tests of Examples 11 and 12 along with the results of the dissolution test of Example 1; and
FIG. 5 shows the results of the dissolution tests of Comparative Examples 5 to 7.

### DETAILED DESCRIPTION OF THE PREFERRED EMBODIMENTS

According to the invention, the solid preparation comprises a two layer structure of a core and a film which covers an outer surface of the core, wherein the core comprises a drug and the film comprises a nonionic water-soluble cellulose ether and a cellulose-based enteric base material.

Examples of the nonionic water-soluble cellulose ether include hydroxypropylmethyl cellulose containing preferably from 19 to 32% by weight of, more preferably from 28 to 30% by weight of a methoxy group, and preferably from 4 to 12% weight of, more preferably from 7 to 12% by weight of a hydroxypropoxy group; methyl cellulose containing preferably from 27.5 to 31.5% by weight of, more preferably from 28 to 31% by weight of a methoxy group; and hydroxypropyl cellulose containing preferably from 53.4 to 77.5% by weight of, more preferably from 60 to 70% by weight of a hydroxypropoxy group. From the standpoint of film formability, hydroxypropylmethyl cellulose is particularly preferred. The nonionic water-soluble cellulose ether may be used singly, or two or more nonionic water-soluble cellulose ethers may be used in combination.

The degrees of substitution can be determined in accordance with the Zeisel-GC method described in J.G. Gobler, E.P. Samsel and G.H. Beaber, Talanta, 9, 474 (1962). The degree of substitution can also be determined by a measurement method of using a gas chromatograph as described in "methyl cellulose", "hydroxypropylmethyl cellulose", or "hydroxypropyl cellulose" in Japanese Standards of Food Additives, Eighth Edition, or a method in accordance with a measurement method for the degree of substitution of methyl cellulose, hydroxypropylmethyl cellulose or hydroxypropyl cellulose specified in the Japanese Pharmacopoeia, Fifteenth Edition.

The viscosity of an aqueous 2% by weight solution of the nonionic water-soluble cellulose ether at 20°C is preferably from 3 to 15 mPa·s, more preferably from 3 to 8 mPa·s from the standpoint of increasing the concentration in a coating solution. The viscosity is measured with an Ubbelohde viscometer in accordance with JIS K2283-1993.

Examples of the cellulose-based enteric base material include hydroxypropylmethyl cellulose acetate succinate, hydroxypropylmethyl cellulose phthalate and carboxyacetyl cellulose. Of these, hydroxypropylmethyl cellulose acetate succinate, which can be dissolved easily and uniformly in a solvent, is particularly preferred. The cellulose-based enteric base material may be used singly, or two or more cellulose-based enteric base materials may be used in combination.

With regards to the content of the substituents of hydroxypropylmethyl cellulose acetate succinate, the content of a methoxy group is preferably from 12 to 28% by weight, more preferably from 20 to 26% by weight, the content of a hydroxypropoxy group is preferably from 4 to 23% by weight, more preferably from 5 to 10% by weigh, the content of an acetyl group is preferably from 2 to 16% by weight, more preferably from 5 to 14% by weight, and the content of a succinoyl group is preferably from 2 to 20% by weight, more preferably from 4 to 18% by weight.

Further, a content ratio of the acetyl group to the succinoyl group is preferably from 1.25 to 5, more preferably from 1.5 to 3.5. When the content ratio is less than 1.25, a sufficient lag time may not be obtained because dissolution occurs on a low pH side. When the content ratio is more than 5, hydrophobicity increases so that dissolution may be prevented.

A mixing weight ratio of the nonionic water-soluble cellulose ether to the cellulose-based enteric base material comprised by the composition for coating solution is from 95:5 to 65:35, preferably from 90:10 to 70:30. When the portion of the nonionic water-soluble cellulose ether is larger than the above range of 95:5 to 65:35, a sufficient lag time cannot be attained. When the portion of the cellulose-based enteric base material is larger than the above range, a solid preparation reaches the large intestine or is excreted without releasing a drug even after the lag time.

The composition for coating solution may optionally comprise a plasticizer. Examples of the plasticizer include glycerin, polyethylene glycols, triethyl citrate, glycerin acetic acid fatty acid esters, triacetin and dibutyl phthalate. As the plasticizer, triethyl citrate and glycerin acetic acid fatty acid esters are preferred. The above plasticizer may be used singly, or two or more plasticizers may be used in combination.

With regard to the content of the plasticizer, when hydroxypropylmethyl cellulose acetate succinate is used as the cellulose-based enteric base material and triethyl citrate is used as the plasticizer, an amount of triethyl citrate is preferably from about 20 to 35 parts by weigh relatively to 100 parts by weight of hydroxypropylmethyl cellulose acetate succinate.

In addition, in general, a surfactant such as sodium lauryl sulfate for enabling to improve the dispersibility of the drug or composition, a colorant, a pigment, a sweetener or the like which is pharmaceutically acceptable, may also be added.

The drug to be used in the invention is not particularly limited insofar as it is orally administrable. Examples of such a drug include chemotherapeutic agents; respiratory stimulants; anticancer drugs; autonomic agents; psychoneurotic agents; local anesthetics; muscle relaxants; pharmaceutical agents affecting digestive organs; drugs for poisoning treatment; hypnotic sedatives; vasodilators; antilipemic agents; nutritional additives for medical purposes, medicinal tonics and substitutes therefore; anticoagulants; pharmaceutical agents for liver; hypoglycermic agents; antihypertensives; anticolitic drugs; peptides; and proteins. In addition, examples of a drug having a bitter taste or the like include antibiotics (such as talampicillin hydrochloride, bacampicillin hydrochloride, cefaclor and erythromycin); antitussive expectorants (such as noscapine hydrochloride, carbetapentane citrate, dextromethorphan hydrobromide, isoaminile citrate and dimemorfan phosphate); antihistamines (such as chlorpheniramine maleate, diphenhydramine hydrochloride and promethazine hydrochloride); antipyretic, analgesic and anti-inflammatory drugs (such as ibuprofen, diclofenac sodium, flufenamic acid, sulpyrine, aspirin and ketoprofen); cardiotonics (such as etilefrine hydrochloride and digitoxin); antiarrhythmic agents (such as propranolol hydrochloride and alprenolol hydrochloride); diuretics (such as caffeine); vasodilators; antilipemic drugs; nutritional additives for medical purposes, medicinal tonics and substitutes therefore; anticoagulants; pharmaceutical agents for liver; hypoglycermic agents; antihypertensives; anticolitic drugs; bronchodilators (such as theophylline); anti-ulcer drugs (such as cimetidine and pirenzepine hydrochloride); sympathetic stimulants (such as dihydrocodeine phosphate and dl-methylephedrine hydrochloride); cardiovascular agents (such as delapril hydrochloride, meclofenoxate hydrochloride and diltiazem hydrochloride); cerebral circulation activators (such as vinpocetine); anxiolytics (such as chlordiazepoxide and diazepam); vitamin preparations (such as fursultiamine, thiamine hydrochloride, calcium pantothenate, ascorbic acid, and tranexamic acid); antimalaria drugs (such as quinine hydrochloride); antidiarrheals (such as loperamide hydrochloride); psychoactive drugs (such as chlorpromazine); and vitamins (such as Vitamin A, Vitamin B1, Vitamin B2, Vitamin B6, Vitamin B12, Vitamin C, Vitamin D, Vitamin E and Vitamin K).

The drug-containing core may comprise a various type of additive usually employed in this field. Such an additive may include an excipient, a binder, a disintegrant, a lubricant, an anticoagulant and a solubilizing agent of a pharmaceutical compound. Examples of the excipient include saccharides such as sucrose, lactose, mannitol and glucose; starches; crystalline cellulose; calcium phosphate; and calcium sulfate. Examples of the binder include polyvinyl alcohol, polyacrylic acid, polymethacrylic acid, polyvinyl pyrrolidone, glucose, sucrose, lactose, maltose, dextrin, sorbitol, mannitol, hydroxyethyl cellulose, hydroxypropylmethyl cellulose, hydroxypropyl cellulose, macrogols, gum arabic, gelatin, agar and starches. Examples of the disintegrant include low-substituted hydroxypropyl cellulose, carmellose or salt thereof, croscarmellose sodium, carboxymethyl starch sodium, crospolyvinyl pyrrolidone, crystalline cellulose, and crystalline cellulose/carmellose sodium. Examples of the lubricant and the anticoagulant include talc, magnesium stearate, calcium stearate, colloidal silica, stearic acid, waxes, hydrogenated oils, polyethylene glycols, and sodium benzoate. In addition, examples of the solubilizing agent for a pharmaceutical compound include organic acids such as fumaric acid, succinic acid, malic acid and adipic acid. The amount of the additive can be determined appropriately depending on the type of the drug or the like.

Accordingly to the invention, examples of the solid preparation include a tablet, a granule, a fine granule and a capsule.

A coating amount on the surface of the core may differ depending on the kind, shape, size or surface condition of the core, or the property of the drug or additive comprised by the core. In general, a coating amount for the tablet may be preferably from 3 to 100% by weight, more preferably from 6 to 12% by weight, and a coating amount for the granule or fine granule may be preferably from 3 to 100% by weight, more preferably from 15 to 30% by weight, in terms of a total coating weight of the nonionic water-soluble cellulose ether and the cellulose-based enteric base material based on the weight of the core. When the coating amount is less than 3% by weight, a sufficient lag time may not be attained. When the coating amount is more than 100% by weight, the coating film may not dissolve completely even in the intestine and the preparation may reach the large intestine or may be excreted without releasing the drug even after the lag time. Accordingly, the coating amounts outside the above ranges may not be preferable.

Next, the method for preparing a solid preparation according to the invention will be described.

In the step of applying a solution of the coating composition to the drug-containing core, a conventionally known coating apparatus can be used. In spray coating, which is typically employed, a pan coating apparatus, a drum type coating apparatus, a fluidized bed coating apparatus, or a stirred fluidized bed coating apparatus may be used. As a spraying device with which such an apparatus is equipped, any of an air spray, an airless spray and a three-fluid spray can be used.

The solvent for dissolving the coating composition is preferably selected from solvents capable of dissolving both the nonionic water-soluble cellulose ether and the cellulose-based enteric base material such as an aqueous 1.0 to 3.0% by weight ammonia solution, a mixed solution of water and ethanol (a preferable weight ratio of water to ethanol is from 90:10 to 10:90) and a mixed solution of water and methanol (a preferable weight ratio of water to methanol is from 90:10 to 10:90). For example, when hydroxypropylmethyl cellulose is used as the nonionic water-soluble cellulose ether and hydroxypropylmethyl cellulose acetate succinate is used as the cellulose-based enteric base material, the mixed solvent of water and ethanol, or the ammonia solution can be used. When both the nonionic water-soluble cellulose ether and the cellulose-based enteric base material have been dissolved, an insoluble dye or the like may be dispersed in the resulting solution.

According to the invention, a method for applying the solution of the coating composition includes, for example, applying a solution obtained by dissolving the coating composition in the solvent to the drug-containing core through spraying or the like by using the above-described coating apparatus. Then, inside of the coating apparatus or outside of the coating apparatus after the resulting core is taken out from the coating apparatus, it is dried by heating or the like to remove the solvent. Consequently, a solid preparation can be prepared.

The solid preparation thus prepared shows that, in the dissolution test specified in the Japanese Pharmacopoeia 15th Edition, a lag time which is a time from start of the test until it starts releasing of the drug in the 1st fluid and purified water is preferably 3 minutes or greater but less than 120 minutes, more preferably 5 minutes or greater but less than 30 minutes. A desired lag time can be attained appropriately by changing the composition or coating amount of the coating solution. Accordingly, it can be used as an alternative for sugar coating which has conventionally shown a time-dependent change in dissolution.

### EXAMPLES

The invention will be described specifically by Examples and Comparative Examples. It should not be construed that the invention is limited to or by them.

### <Example 1>

Uncoated tablets containing riboflavin as a drug were prepared by mixing, in powder form, 2 parts by weight of riboflavin (product of Tokyo Tanabe Pharma Corporation), 90 parts by weight of lactose ("Dilactose S", product of Freund Corporation), 8 parts by weight of low-substituted hydroxypropyl cellulose (product of Shin-Etsu Chemical Co., Ltd., the degree of hydroxypropyl substitution: 11% by weight) and 0.5 parts by weight of magnesium stearate, and tableting the resulting mixture with a rotary tablet press ("Virgo", product of Kikusui Seisakusho) under the conditions of a tablet diameter of 8 mm, a tableting pressure of 1 t, and a tableting pre-pressure of 0.3 t and number of revolutions of 20 rpm to allow a weight per tablet to be 200 mg.

A coating solution was prepared by using hydroxypropylmethyl cellulose (HPMC) (product of Shin-Etsu Chemical Co., ltd., the degree of methoxy substitution: 29% by weight, the degree of hydroxypropoxy substitution: 10% by weight, a viscosity of an aqueous 2% by weight solution thereof at 20°C: 6.0 mPa·s as measured using an Ubbelohde viscometer specified in JIS K2283-1993) as a water-soluble cellulose ether and hydroxypropylmethyl cellulose acetate succinate (HPMCAS) (product of Shin-Etsu Chemical Co., Ltd., a methoxy group: 29% by weight, a hydroxypropoxy group: 10% by weight, an acetyl group: 9% by weight, a succinoyl group: 11% by weight) as a cellulose-based enteric base material in accordance with the formulation shown in Table 1 to allow a weight ratio of HPMC to PMCAS to be 90:10.

The uncoated tablets were coated with the coating solution thus obtained under the following conditions until the total weight of HPMC and HPMCAS reached 6% by weight in total relatively to the weight of the uncoated tablet.

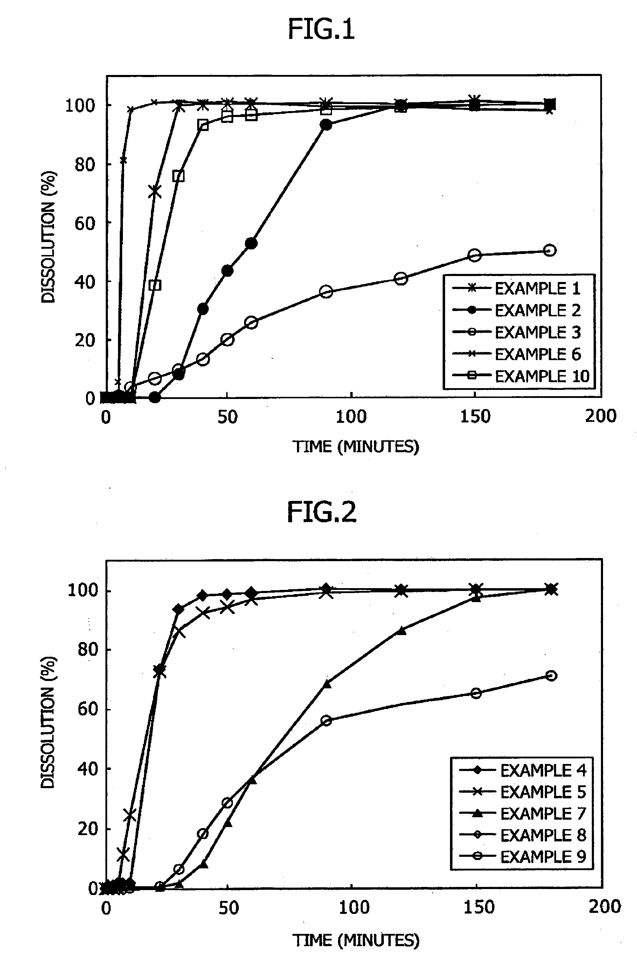

The dissolution test in pure water was performed on the coated tablets according to the "Dissolution Test" of the Japanese Pharmacopoeia, 15th Edition and evaluation results are shown in FIG. 1.

Sampling in the dissolution test was conducted 1 minute, 3 minutes, 5 minutes, 7 minutes, 10 minutes, 20 minutes, 30 minutes, 60 minutes, 90 minutes, 120 minutes, 150 minutes, and 180 minutes after the dissolution test was started and the dissolution start time was determined as a lag time. The lag time is shown in Table 1.
Coating Conditions:
Apparatus: perforated pan coater (inner diameter: 30 cm)
Charged amount: 1 kg
Inlet air temperature: 80°C
Outlet air temperature: from 48 to 51°C
Inlet air flow rate: 1 m³/min
Rotating speed of the pan: 18 rpm
Spray rate: 6 g/min
Spray air pressure: 150 kPa

### <Example 2>

The coating solution obtained in the same manner as in Example 1 was applied to the uncoated tablets until the total amount of HPMC and HPMCAS reached 10% by weight relatively to the weight of the uncoated tablet. The results of the dissolution test performed on the coated tablets by using pure water are shown in FIG. 1, and the lag time is shown in Table 1.

### <Example 3>

The coating solution was prepared in the same manner as in Example 1 except that the weight ratio of HPMC to HPMCAS was changed to 70:30.

In the same manner as in Example 1, the coating solution was applied to the uncoated tablets until the total amount of HPMC and HPMCAS reached 3% by weight relatively to the weight of the uncoated tablet. The results of the dissolution test performed on the coated tablets by using pure water are shown in FIG. 1, and the lag time is shown in Table 1.

### <Example 4>

A coating solution was prepared by using HPMC (product of Shin-Etsu Chemical Co., Ltd., the degree of methoxy substitution: 29% by weight, the degree of hydroxypropoxy substitution: 10% by weight, a viscosity of an aqueous 2.0% by weight solution at 20°C as measured using an Ubbelohde viscometer specified in JIS K2283-1993: 6.0 mPa·s) as a water-soluble cellulose ether and HPMCAS (product of Shin-Etsu Chemical Co., Ltd., the degree of methoxy substitution: 29% by weight, the degree of hydroxypropoxy substitution: 10% by weight, the degree of acetyl substitution: 9% by weight, the degree of succinoyl substitution: 11 % by weight) as a cellulose-based enteric base material in accordance with the formulation shown in Table 1 to allow a weight ratio of HPMC to HPMCAS to be 90: 10, wherein a mixed solvent, a 20:80 (weight ratio) mixture of water and ethanol, was used.

In the same manner as in Example 1, the coating solution thus obtained was applied to the uncoated tablets until the total amount of HPMC and HPMCAS reached 10% by weight relatively to the weight of the uncoated tablet. The results of the dissolution test performed on the coated tablets using pure water are shown in FIG. 2, and the lag time is shown in Table 1.

### <Example 5>

The coating solution was prepared in the same manner as in Example 4 except that the weight ratio of HPMC to HPMCAS was changed to 70:30.

In the same manner as in Example 1, the coating solution was applied to the uncoated tablets until the total amount of HPMC and HPMCAS reached 6% by weight relatively to the weight of the uncoated tablet. The results of the dissolution test performed on the coated tablets using pure water are shown in FIG. 2, and the lag time is shown in Table 1.

### <Example 6>

A coating solution was prepared by using HPMC (product of Shin-Etsu Chemical Co., Ltd., the degree of methoxy substitution: 29% by weight, the degree of hydroxypropoxy substitution: 10% by weight, a viscosity of an aqueous 2.0% by weight solution at 20°C as measured using an Ubbelohde viscometer specified in JIS K2283-1993: 6.0 mPa·s) as a water-soluble cellulose ether and HPMCAS (product of Shin-Etsu Chemical Co., Ltd., the degree of methoxy substitution: 29% by weight, the degree of hydroxypropoxy substitution: 10% by weight, the degree of acetyl substitution: 9% by weight, the degree of succinoyl substitution: 11 % by weight) as a cellulose-based enteric base material in accordance with the formulation shown in Table 1 to allow a weight ratio of HPMC to HPMCAS to be 90: 10, wherein an aqueous 4% by weight solution of sodium hydroxide and purified water were used as a solvent.

In the same manner as in Example 1, the coating solution thus obtained was applied to the uncoated tablets until the total amount of HPMC and HPMCAS reached 10% by weight relatively to the weight of the uncoated tablet. The results of the dissolution test performed on the coated tablets by using pure water are shown in FIG. 1, and the lag time is shown in Table 1.

### <Example 7>

A coating solution was prepared by using methyl cellulose (MC) (product of Shin-Etsu Chemical Co., Ltd., the degree of methyl substitution: 28% by weight, a viscosity of an aqueous 2.0% by weight solution at 20°C as measured using an Ubbelohde viscometer specified in JIS K2283-1993: 4.0 mPa·s) as a water-soluble cellulose ether and HPMCAS (product of Shin-Etsu Chemical Co., Ltd., degree of methoxy substitution: 29% by weight, the degree of hydroxypropoxy substitution: 10% by weight, the degree of acetyl substitution: 9% by weight, the degree of succinoyl substitution: 11% by weight) as a cellulose-based enteric base material in accordance with the formulation shown in Table 1 to allow a weight ratio of MC to HPMCAS to be 90:10, wherein a mixed solvent, 20:80 (weight ratio) mixture of water and ethanol, was used.

In the same manner as in Example 1, the coating solution thus obtained was applied to the uncoated tablets until the total amount of MC and HPMCAS reached 6% by weight relatively to the weight of the uncoated tablet. The results of the dissolution test performed on the coated tablets by using pure water are shown in FIG. 2, and the lag time is shown in Table 1.

### <Example 8>

A coating solution was prepared by using hydroxypropyl cellulose (HPC) (product of Nippon Soda Co., Ltd., the degree of hydroxypropyl substitution: 80% by weight, a viscosity of an aqueous 2.0% by weight solution at 20°C as measured using an Ubbelohde viscometer specified in JIS K2283-1993: 4.5 mPa·s) as a water-soluble cellulose ether and HPMCAS (product of Shin-Etsu Chemical Co., Ltd., degree of methoxy substitution: 29% by weight, the degree of hydroxypropoxy substitution: 10% by weight, the degree of acetyl substitution: 9% by weight, and the degree of succinoyl substitution: 11% by weight) as a cellulose-based enteric base material in accordance with the formulation shown in Table 1 to allow a weight ratio of HPC to HPMCAS to be 90:10, wherein a mixed solvent, a 20:80 (weight ratio) mixture of water and ethanol, was used.

In the same manner as in Example 1, the coating solution thus obtained was applied to the uncoated tablets until the total amount of HPC and HPMCAS reached 6% by weight relatively to the weight of the uncoated tablet. The results of the dissolution test performed on the coated tablets by using pure water are shown in FIG. 2 and the lag time is shown in Table 1. The coated tablets showed the same dissolution behavior as that of the coated tablets obtained in Example 4.

### <Example 9>

A coating solution was prepared by using HPMC (product of Shin-Etsu Chemical Co., Ltd., the degree of methoxy substitution: 29% by weight, the degree of hydroxypropoxy substitution: 10% by weight, a viscosity of an aqueous 2.0% by weight solution at 20°C as measured using an Ubbelohde viscometer specified in JIS K2283-1993: 6.0 mPa·s) as a water-soluble cellulose ether and hydroxypropylmethyl cellulose phthalate (HPMCP) (product of Shin-Etsu Chemical Co., Ltd., degree of methoxy substitution: 6% by weight, the degree of hydroxypropoxy substitution: 20% by weight, and the degree of carboxybenzoyl substitution: 33% by weight) as a cellulose-based enteric base material in accordance with the formulation shown in Table 1 to allow a weight ratio of HPMC to HPMCP to be 70:30, wherein a mixed solvent, a 20:80 (weight ratio) mixture of water and ethanol, was used.

In the same manner as in Example 1, the coating solution thus obtained was applied to the uncoated tablets until the total amount of HPMC and HPMCP reached 10% by weight relatively to the weight of the uncoated tablet. The results of the dissolution test on the coated tablets by using pure water are shown in FIG. 2 and the lag time is shown in Table 1.

### <Example 10>

A coating solution prepared in the same manner as in Example 1 was applied to uncoated tablets prepared in the same manner as in Example 1 until the total amount reached 6% by weight relatively to the weight of the uncoated tablet. The dissolution test in the first liquid (pH 1.2) of the Japanese Pharmacopoeia was performed on the coated tablets in accordance with the "Dissolution Test" of the Japanese Pharmacopoeia 15th Edition. The evaluation results are shown in FIG. 1 and the lag time is shown in Table 1. It was found from the results that the pH of the dissolution fluid did not provide a large change in lag time.

### <Comparative Example 1>

In the same manner as in Example 1 except that the weight ratio of HPMC to HPMCAS was changed to 98:2, a coating solution was prepared.

In the same manner as in Example 1, the resulting coating solution was applied to the uncoated tablets until the total amount of HPMC and HPMCAS became 6% by weight relatively to the weight of the uncoated tablet. The results of the dissolution test performed on the coated tablets by using pure water are shown in FIG. 1 and the lag time is described in Table 1. The drug dissolved without a lag time.

### <Comparative Example 2>

A coating solution was prepared by using HPMC (product of Shin-Etsu Chemical Co., Ltd., the degree of methoxy substitution: 29% by weight, the degree of hydroxypropoxy substitution: 10% by weight, a viscosity of an aqueous 2.0% by weight solution at 20°C as measured using an Ubbelohde viscometer specified in JIS K2283-1993: 6.0 mPa·s) as a water-soluble cellulose ether and HPMCAS (product of Shin-Etsu Chemical Co., Ltd., degree of methoxy substitution: 29% by weight, the degree of hydroxypropoxy substitution: 10% by weight, the degree of acetyl substitution: 9% by weight, and the degree of succinoyl substitution: 11 % by weight) as a cellulose-based enteric base material in accordance with the formulation shown in Table 1 to allow a weight ratio of HPMC to HPMCAS to be 60:40.

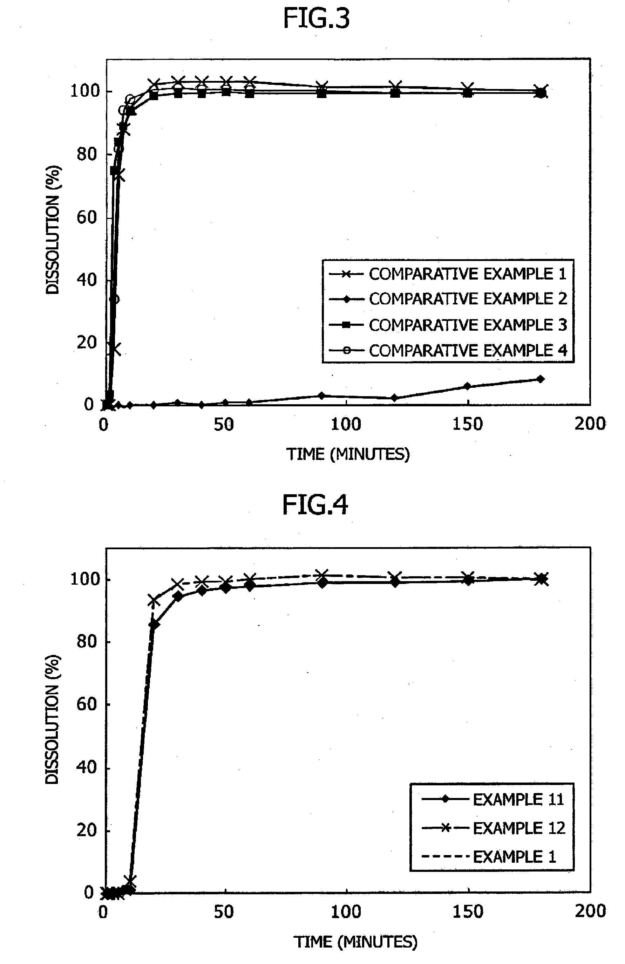

In the same manner as in Example 1, the coating solution thus obtained was applied to the uncoated tablets until the total amount of HPMC and HPMCAS reached 6% by weight relatively to the weight of the uncoated tablet. The results of the dissolution test performed on the coated tablets by using pure water are shown in FIG. 3 and the lag time is shown in Table 1.

Sustained release of the drug occurred without lag time and the drug did not dissolve completely in the stomach.

### <Comparative Example 3>

An aqueous dispersion for coating was prepared by using HPMC (product of Shin-Etsu Chemical Co., Ltd., the degree of methoxy substitution: 29% by weight, the degree of hydroxypropoxy substitution:10% by weight, a viscosity of an aqueous 2.0% by weight solution at 20°C as measured using an Ubbelohde viscometer specified in JIS K2283-1993: 6.0 mPa·s) as a water-soluble cellulose ether and HPMCAS (product of Shin-Etsu Chemical Co., Ltd., degree of methoxy substitution: 29% by weight, the degree of hydroxypropoxy substitution: 10% by weight, the degree of acetyl substitution: 9% by weight, and the degree of succinoyl substitution: 11% by weight) as a cellulose-based enteric base material in addition of 0.59 part by weight of triethyl citrate in accordance with the formulation shown in Table 1 to allow a weight ratio of HPMC to HPMCAS to be 70:30.

In the same manner as in Example 1, the aqueous dispersion thus obtained was applied to the uncoated tablets until the total amount of HPMC and HPMCAS reached 3% by weight relatively to the weight of the uncoated tablet. The results of the dissolution test performed on the coated tablets by using pure water are shown in FIG. 3 and the lag time is shown in Table 1.

Compared with the results of Example 3, dissolution of the drug occurred one minute after the dissolution test was started. Thus, dissolution occurred without lag time.

### <Comparative Example 4>

An aqueous dispersion for coating was prepared by using HPMC (product of Shin-Etsu Chemical Co., Ltd., the degree of methoxy substitution: 29% by weight, the degree of hydroxypropoxy substitution: 10% by weight, a viscosity of an aqueous 2.0% by weight solution at 20°C as measured using an Ubbelohde viscometer specified in JIS K2283-1993: 6.0 mPa·s) as a water-soluble cellulose ether and a methacrylic acid copolymer ("Eudragit", trade mark; product of Evonik Industries, solid content of the methacrylic acid copolymer: 30% by weight) as an enteric base material in accordance with the formulation shown in Table 1 to allow a weight ratio of HPMC to the solid content of methacrylic acid copolymer to be 90:10.

In the same manner as in Example 1, the resulting aqueous dispersion was applied to the uncoated tablets until the total amount of HPMC and the solid content of the methacrylic acid copolymer reached 6% by weight relatively to the weight of the uncoated tablet.

In the same manner as in Example 1, the dissolution test in pure water was performed on the coated tablets in accordance with the dissolution test method of the Japanese Pharmacopoeia. The evaluation results are shown in FIG. 3 and the lag time is shown in Table 1.

Compared with the results of Example 1, dissolution of the drug was observed one minute after the dissolution test was started. Thus, the dissolution occurred without a lag time.

**Table 1**

| | water-soluble cellulose ether (pbw) | | | enteric base material (pbw) | | | solvent (parts by weight pbw) | | | | plasticizer (pbw) triethyl citrate | sum | amount of coating | lag time (minute) | dissolution after lag time |
|---|---|---|---|---|---|---|---|---|---|---|---|---|---|---|---|
| | HPMC | MC | HPC | HPMCAS | HPMCP | methacrylic acid copolymer | 10wt% ammonia water | aq 4wt% NaOH solution | C₂H₅OH | purified water | | (pbw) | (%) (*1) | | |
| Example 1 | 6.3 | - | - | 0.7 | - | - | 0.13 | - | - | 92.87 | - | 100 | 6 | 10 | immediate dissolution |
| Example 2 | 6.3 | - | - | 0.7 | - | - | 0.13 | - | - | 92.87 | - | 100 | 10 | 20 | immediate dissolution |
| Example 3 | 4.9 | - | - | 2.1 | - | - | 0.40 | - | - | 92.60 | - | 100 | 3 | 7 | immediate dissolution |
| Example 4 | 6.3 | - | - | 0.7 | - | - | - | - | 74.40 | 18.60 | - | 100 | 10 | 10 | immediate dissolution |
| Example 5 | 4.9 | - | - | 2.1 | - | - | - | - | 74.40 | 18.60 | - | 100 | 6 | 5 | immediate dissolution |
| Example 6 | 6.3 | - | - | 0.7 | - | - | - | 7.80 | - | 85.20 | - | 100 | 10 | 5 | immediate dissolution |
| Example 7 | - | 6.3 | - | 0.7 | - | - | - | - | 74.40 | 18.60 | - | 100 | 6 | 30 | immediate dissolution |
| Example 8 | - | - | 6.3 | 0.7 | - | - | - | - | 74.40 | 18.60 | - | 100 | 6 | 10 | immediate dissolution |
| Example 9 | 4.9 | - | - | - | 2.1 | - | - | - | 74.40 | 18.60 | - | 100 | 10 | 20 | immediate dissolution |
| Example10 | 6.3 | - | - | 0.7 | - | - | 0.13 | - | - | 92.87 | - | 100 | 6 | 10 | immediate dissolution |
| Comp Ex 1 | 6.86 | - | - | 0.14 | - | - | 0.03 | - | - | 92.97 | - | 100 | 6 | none | - |
| Comp Ex 2 | 4.2 | - | - | 2.8 | - | - | 0.52 | - | - | 92.48 | - | 100 | 6 | none | - |
| Comp Ex 3 | 4.9 | - | - | 2.1 | - | - | - | - | - | 92.41 | 0.59 | 100 | 3 | 1 | - |
| Comp Ex 4 | 6.3 | - | - | - | - | 2.3 | - | - | - | 91.40 | - | 100 | 6 | 1 | - |

| | | | | | | | | | | | | | | | |
|---|---|---|---|---|---|---|---|---|---|---|---|---|---|---|---|
| *1 The amount of coating is shown by wt% (% by weight) relative to the weight of uncoated tablet | | | | | | | | | | | | | | | |

<Examples 11 and 12>

In Example 11, the coated tablets obtained in Example 1 were placed in a plastic bottle and stored in a drier having a humidity-controlling-function and being kept constant at 40°C and 75%RH for one month without sealing the bottle,

In Example 12, separately from Example 11, the coated tablets obtained in Example 1 were placed in a plastic bottle, then sealed hermetically, and stored in a drier kept constant at 50°C for one month.

The dissolution test of two kinds of the coated tablets subjected to the storage test was conducted in pure water in the same manner as in Example 1 and the results are shown in FIG. 4. None of these two kinds of the coated tablets showed a time-dependent change in dissolution.

### <Comparative Examples 5 to 7>

An aqueous dispersion for coating was prepared by heating to 60°C a mixture of 66.7 parts by weight of purified sucrose, 33.3 parts by weight of purified water, 2.5 parts by weight of gum arabic as a binder, 0.6 part by weight of gelatin, 36.7 parts by weight of calcium carbonate and 30 parts by weight of talc to dissolve the purified sucrose.

The aqueous dispersion for coating was applied to the uncoated tablets obtained in Example 1 under the following conditions until the weight of the coating layer increased by 80% by weight and preparation of sugar coated tablets was completed.
Coating conditions:
Apparatus: perforated pan coater (inner diameter: 50 cm)
Charged amount: 3.5 kg
Inlet air temperature: 60°C and then 40°C
Outlet air temperature: from 48 to 51°C
Inlet air flow rate: 2.5 m³/min
Rotating speed of the pan: 18 rpm
Spray air pressure: 50 kPa, 60 L/min

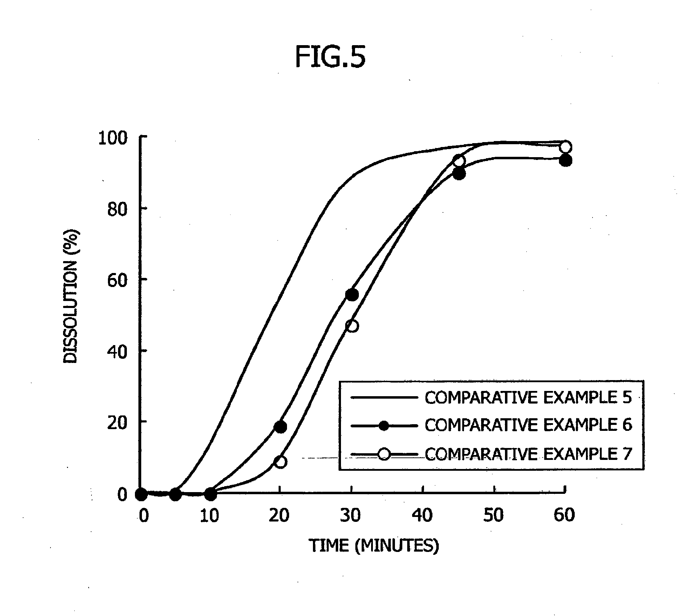

In Comparative Example 5, the dissolution test of the sugar-coated tablets by using pure water was performed in the same manner as in Example 1 and the results are shown in FIG. 5. In Comparative Example 6, the sugar-coated tablets thus obtained were placed in a plastic bottle and stored in a drier having a humidity controlling function and being kept constant at 40°C and 75%RH for one month without sealing the bottle. In Comparative Example 7, separately from Example 6, the sugar-coated tablets thus obtained were placed in another plastic bottle, then sealed hermetically, and stored in a drier kept constant at 50°C for one month. The dissolution test of the resulting two kinds of the sugar-coated tablets subjected to respective storage tests was conducted using pure water in the same manner as in Example 1 and the results are shown in FIG. 5, together with the results of Comparative Example 5.

With respect to both the coated tablets obtained in Example 11 which had been stored at 40°C and 75%RH without sealing the bottle and the coated tablets obtained in Example 12 which had been stored at constant temperature of 50°C while hermetic sealing, no change occurred in lag time between before storage and after storage. In Comparative Examples 6 and 7, however, the sugar-coated tablets had insufficient stability because a change in lag time occurred after the storage test.

## Claims

1. A coating solution comprising :
a coating composition comprising at least a nonionic water-soluble cellulose ether and a cellulose-based enteric base material at a weight ratio of from 95:5 to 65:35, and
a solvent capable of dissolving both the nonionic water-soluble cellulose ether and the cellulose-based enteric base material,
wherein the water-soluble cellulose ether is selected from the group consisting of hydroxypropylmethylcellulose, methylcellulose, hydroxypropylcellulose, and combinations thereof, and wherein the cellulose-based enteric base material is selected from the group consisting of hydroxypropylmethylcellulose acetate succinate, hydroxypropylmethylcellulose phthalate and combinations thereof.

2. A solid preparation comprising at least a drug-containing core and the coating composition as claimed in Claim 1, which covers the core, wherein the preparation can permit prompt dissolution of the drug in a stomach after a lag time.

3. A method for preparing a solid preparation, comprising the steps of:
applying, to a drug-containing core, a solution of the coating composition as claimed in Claim 1 in a solvent; and
drying to remove the solvent.

4. The method according to Claim 3, wherein the solvent is an aqueous ammonia solution.

## Patentansprüche

1. Beschichtungslösung umfassend:
eine Beschichtungszusammensetzung umfassend wenigstens einen nicht-ionischen, wasserlöslichen Celluloseether und ein Cellulose-basiertes enterisches Basismaterial in einem Gewichtsverhältnis von 95:5 bis 65:35, und
ein Lösungsmittel, welches fähig ist, beide, den nicht-ionischen, wasserlöslichen Celluloseether und das Cellulose-basierte enterische Basismaterial aufzulösen,
wobei der wasserlösliche Celluloseether ausgewählt ist aus der Gruppe bestehend aus Hydroxypropylmethylcellulose, Methylcellulose, Hydroxypropylcellulose, und Kombinationen davon, und wobei das Cellulose-basierte enterische Basismaterial ausgewählt ist aus der Gruppe bestehend aus Hydroxypropylmethylcelluloseacetatsuccinat, Hydroxypropylmethylcellulosephtalat und Kombinationen davon.

2. Feste Zubereitung umfassend wenigstens einen Arzneimittel-enthaltenden Kern und die Beschichtungszusammensetzung wie in Anspruch 1 beansprucht, welche den Kern bedeckt, wobei die Zubereitung das unverzügliche Auflösen des Arzneimittels in einem Magen nach einer Verzögerungszeit erlauben kann.

3. Verfahren zur Herstellung einer festen Zubereitung, umfassend die Schritte:
Auftragen einer Lösung der Beschichtungszusammensetzung wie in Anspruch 1 beansprucht in einem Lösungsmittel auf einen Arzneimittel-enthaltenden Kern; und
Trocknen, um das Lösungsmittel zu entfernen.

4. Verfahren nach Anspruch 3, wobei das Lösungsmittel eine wässrige AmmoniakLösung ist.

## Revendications

1. Solution de revêtement, comprenant :
une composition de revêtement comprenant au moins un éther cellulosique hydrosoluble non ionique et une substance entérique de base à base de cellulose à un rapport pondéral de 95:5 à 65:35, et
un solvant capable de dissoudre à la fois l'éther cellulosique hydrosoluble non ionique et la substance entérique de base à base de cellulose,
dans laquelle l'éther cellulosique hydrosoluble est choisi dans le groupe constitué par l'hydroxypropylméthylcellulose, la méthylcellulose, l'hydroxypropylcellulose et leurs combinaisons, et dans laquelle la substance entérique de base à base de cellulose est choisie dans le groupe constitué par le succinate d'acétate d'hydroxypropylméthylcellulose, le phtalate d'hydroxypropylméthylcellulose et leurs combinaisons.

2. Préparation solide comprenant au moins un noyau contenant un médicament et une composition de revêtement selon la revendication 1 qui recouvre le noyau, dans laquelle la préparation peut permettre une dissolution rapide du médicament dans un estomac après un temps de latence.

3. Procédé de préparation d'une préparation solide, comprenant les étapes suivantes :
l'application, sur un noyau contenant un médicament, d'une solution de la composition de revêtement selon la revendication 1 dans un solvant ; et
un séchage pour éliminer le solvant.

4. Procédé selon la revendication 3, dans lequel le solvant est une solution aqueuse d'ammoniaque.
